(19) Europäisches Patentamt
European Patent Office
Office européen des brevets

(11) Publication number: 0 272 026 B1

(12) EUROPEAN PATENT SPECIFICATION

(45) Date of publication of patent specification: 03.02.93

(51) Int. Cl.5: C12N 9/80, C12P 7/40, //(C12P7/40,C12R1:02)

(21) Application number: 87310710.6

(22) Date of filing: 04.12.87

(54) **Process for the decomposition of acrylamide.**

(30) Priority: 16.12.86 GB 8630012

(43) Date of publication of application: 22.06.88 Bulletin 88/25

(45) Publication of the grant of the patent: 03.02.93 Bulletin 93/05

(84) Designated Contracting States: BE DE ES FR GB IT NL

(56) References cited:
EP-A- 0 272 025
US-A- 4 532 214

CHEMICAL ABSTRACTS, vol. 91, no. 11, 10th September 1979, page 610, no. 89550d, Columbus, Ohio, US; && JP-A-79 46 887 (NITTO CHEMICAL INDUSTRY CO., LTD) 13-04-1979

AGRICULTURAL AND BIOLOGICAL CHEMISTY, vol. 46, no. 5, 1982, pages 1175-1181, Tokyo, JP; Y. ASANO et al.: "Purification and characterization of amidase which participates in nitrile degradation"

(73) Proprietor: IMPERIAL CHEMICAL INDUSTRIES PLC
Imperial Chemical House, Millbank
London SW1P 3JF(GB)

(72) Inventor: Carver, Mark Andrew
Hallswood 503 Yarm Road&Eaglescliffe
Stockton-on-Tees Cleveland TS16 9BE(GB)
Inventor: Hinton, John
24 Eagle Park Marton
Middlesborough Cleveland TS8 9NT(GB)

(74) Representative: Locke, Timothy John et al
ICI Group Patents Services Dept. PO Box 6
Shire Park Bessemer Road
Welwyn Garden City Herts, AL7 1HD(GB)

EP 0 272 026 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for the decomposition of acrylamide, to a method for the production of the enzyme amidase (acrylamide amidohydrolase EC No. 3-5-1-4) used in the decomposition of acrylamide and to a process for the production of acrylic acid or a salt or ester thereof by decomposition of acrylamide.

Polyacrylamide polymers, i.e. homo- and hetero- polymers of acrylamide, are widely used as flocculants in the potable water industry, sewage treatment, paper manufacture and mining. However their utility is restricted and they cannot for instance be used in connection with foodstuffs because they are generally contaminated with unreacted acrylamide monomer which is a cumulative neurotoxin and a carcinogen. At present polyacrylamides are generally allowed in USA to contain up to 500 ppm of unreacted acrylamide and this limit is likely to be introduced elsewhere. This level of unreacted monomer can be achieved by use of longer polymerisation times and by a "heat treating" process. Such treatments increase cost of manufacture and reduce the efficiency of the polymers produced by causing branching in these polymers. Linear polymers have a higher flocculation efficiency and are preferred. There is a need for a reliable process for reducing the level of unreacted monomer in polyacrylamides without damage to the polymer to 500 ppm and preferably to even lower levels (5-10 ppm or lower). If the unreacted monomer present could be reduced reliably to very low levels (1-5 ppm or less) polyacrylamides could be considered for uses from which they are presently excluded, e.g. in the manufacture of food contact materials in in direct contact with food.

Japanese Patent Publication No. 79011389 (corresponding to Japanese kokai No. 53086078) discloses a process for the decomposition of acrylamide monomer, e.g. in waste water or in polyacrylamides by contacting with an intracellular enzyme of Brevibacterium ammoniogenes preferably of strains ATCC 1641, ATCC 6871 and ATCC 6872. However, despite being known for a number of years, this process does not appear to have been used commercially to any significant extent. Moreover Brevibacterium ammoniogenes enzyme works poorly in polyacrylamide latex systems.

It is desirable that amidase enzymes having increased activity should become readily available commercially.

According to the present invention we provide a method for the production of an amidase enzyme of increased activity wherein microbial cells or extracts containing the enzyme are heated to a temperature in the range 40° to 80°C for a sufficient period and under such conditions that a significant increase in amidase activity is induced. Microbial cells and extracts having increased amidase activity produced by the method of the invention are also included in the scope of the invention.

Further according to the invention we provide a process for the decomposition of acrylamide in a medium containing it in which the medium is contacted with an enzyme capable of decomposing acrylamide under conditions suitable for the enzyme to decompose the acrylamide wherein the enzyme is an amidase enzyme which has been heated to a temperature in the range 40° to 80°C for a sufficient period under suitable conditions to increase the activity of the enzyme.

Further according to the invention we provide a process for the production of acrylic acid or a salt or ester thereof in which a medium containing acrylamide is contacted with an enzyme capable of decomposing acrylamide to acrylic acid under conditions suitable for the enzyme to decompose the acrylamide to acrylic acid or a salt or ester thereof wherein the enzyme is an amidase enzyme which has been heated to a temperature in the range 40° to 80°C for a sufficient period under suitable conditions to increase the activity of the enzyme.

Amidases are part of the nitrogen assimilation apparatus of many cells grown under conditions where the sole source of nitrogen is an aliphatic amide.

Thus the metabolic role of amidase is to release ammonia from said amide, e.g. by the following reaction:-

$$CH_3-\overset{\overset{O}{\|}}{C}-NH_2 \quad + \quad H_2O \quad \text{----->} \quad CH_3-\overset{\overset{O}{\|}}{C}-OH \quad + \quad NH_3$$

The ammonia released is then assimilated into protein biosynthesis. The aliphatic acid produced may or may not be assimilated dependent upon the microorganism type.

Amidases can also catalyse the conversion of acrylamide to acrylic acid by the following reaction:-

$$CH_2 = CH-C(=O)-NH_2 \quad + \quad H_2O \quad ------> \quad CH_2 = CH - C(=O) - OH \quad + \quad NH_3$$

In the presence of appropriate salts or alcohols the acrylic acid produced can be converted to its salt or ester.

The decomposition process of the invention can be used to decompose acrylamide in any medium in which it occurs. It is particularly useful for decomposing unreacted acrylamide present in polyacrylamide polymers and acrylamide present in waste waters, e.g. waste water from a process for the production of polyacrylamides. Polyacrylamides are produced as polymers of three types i.e. solution, dry and suspension polymers.

Polyacrylamides are produced as polymers of three chemical types. These are cationic, anionic and nonionic polymers wherein acrylamides may be copolymerised with other monomers, e.g. acrylic acid. These polymers may be manufactured by one of the following three basic technologies:-

Solution polymerisation wherein monomers are polymerised in aqueous solution to produce a gel-type product;

Dry polymers which are polymers produced as above but which are subsequently heat dehydrated prior to use;

Latex or suspension polymerisation wherein a solution of monomers in water is admixed with detergents and a non-aqueous low odour paraffinic solvent to form a stable suspension of aqueous droplets within which beads of polymers are formed by addition of a water soluble polymerisation initiator to the system.

Latex or suspension polymers form the largest group of polyacrylamides in terms of market share.

The amidase enzyme used in the decomposition and acrylic acid production processes of the invention may be present in any suitable form in whole microorganism cells or as a crude or purified enzyme extract. The enzyme can be introduced to the processes in whole cells in the culture produced initially in the method of the invention or in a medium produced after only partial separation of water and other components from such a culture. Generally however it is preferred that the cells should be separated from the culture before being used in the processes.

When cells or cell free extracts containing amidase produced by the method of the invention are heated to temperatures in the range 40° to 80°C we have found that, most unusually, the amidase activity is irreversibly increased by 1.5 to 35 times dependent upon the enzyme preparation. This effect is greatest at temperatures in the range 55° to 65°C, especially in the range 58° to 62°C, at pHs in the range 4 to 9, especially at pHs between 6 and 7 and at protein concentrations in the range 0.1 to 200 mg/ml. Under these conditions little significant denaturation of amidase takes place.

Heating suitably takes place for a period in the range 20 minutes to 10 hours, especially 30 minutes to 180 minutes in some instances. To produce the increased activity the cells or cell-free enzyme can be heated immediately after they have been produced by the method of the invention or at some later time. The method of the invention can usefully be carried out either as described above or as a similar process having three steps, i.e. a first fermentation step in which cells having induced amidase are grown in a fermenter, a second heat shock step in which cells are subjected to heating to a temperature in the ranges described above and a third separation step in which a precipitate containing debris is separated from a supernatant liquid containing the enzyme.

The amidase enzyme the activity of which is increased by the method of the invention may be contained in whole microbial cells or in crude or purified extracts thereof. The enzyme may be derived from any suitable microbial source. Suitable bacterial sources include strains of the genera Brevibacterium, Pseudomonas, Alcaligenes, Arthobacter, Corynebacterium, Mycobacterium, Lactobacillus, Bacillus, Micrococcus, Nocardia and Streptomyces. Suitable yeasts and fungi include strains of Fusarium and Aspergillus.

Very suitable sources are strains of the species Methylophilus methylotrophus in which the enzyme has been induced as described in our co-pending British Patent Application No. 8630029 (ICI Case B 34138) by cultivation in a medium containing appropriate nutrients and an amide under conditions such that the enzyme is induced in the bacterium.

The species Methylophilus methylotrophus (formerly named Pseudomonas methylotropha), strains of which may be heated in the method of the invention, is described in our UK Patent Specification No. 1370892. Strains of this species suitable for use in the method of the invention have been deposited at the following 3 culture collections from which cultures are available:-

1. The National Collection of Industrial Bacteria (NCIB), Torrey Research Station, PO Box 31, 135 Abbey Road, Aberdeen AB9 8DG, Scotland, UK;

2. The Agricultural Research Culture Collection (NRRL), 1815 North University Street, Peoria, Illinois 61604, USA; and

3. The Fermentation Research Institute (FRI), Agency of Industrial Science and Technology, Ministry of International Trade and Industry, 1 - 3 Higashi 1-Chome, Yatabe-machi, Tsukuba-gun, Ibaragi-ken, Japan.

The corresponding accession numbers assigned to the strains deposited at these collections are as follows:-

NCIB 10508 - 10515 & 10592 - 10596, all inclusive;
NRRL B 5352 - B 5364 inclusive; and
FRI 1215 - 1227 inclusive.

A preferred strain in which amidase may be induced is Methylophilus methylotrophus strain AS-1 (NCIB 10515) which may safely be used in treating, e.g. polyacrylamide polymers intended for use in connection with foods. The species Methylophilus methylotrophus and the above strains, particularly strain AS-1, have become widely known and are mentioned in numerous publications both by us and others. In addition to the deposits mentioned above cultures of them are also held in a number of Universities and other laboratories in the UK and in other countries. Also very suitable for use in the method of the invention is strain NCIB 11585, available from NCIB, the production of which by the genetic modification of strain NCIB 10515 is described in our European Patent Specification 35831 B and a number of other publications.

Methylophilus methylotrophus amidase properties

| | | | |
|---|---|---|---|
| Physiological status | Inducible | | |
| Location | Cytoplasmic | | |
| Protein | Soluble | | |
| | Acidic | Isoelectric point | 4.2 |
| | Tetrameric | | |
| | Native MW | (Gel permeation) | 155,000 |
| | Monomer MW | (SDS page) | 38,000 |
| Properties | pH optimum | Acetamide | 5 |
| | | Acrylamide | 8 |
| | Temperature optimum | | 60°C |
| | Km | Acetamide | 0.2 mM |
| | | Acrylamide | 10 mM |
| | Activities | Acyl amidase | |
| | | Acyl transferase | |
| | Substrate | $C_1 < C_2 < C_3 > C_4 > C_5$ | |
| | | Acetamide < Acrylamide > propionamide | |

Amidase can be induced in Methylophilus methylotrophus when it is cultivated aerobically in a medium containing sources of carbon, nitrogen, phosphorus and other appropriate nutrients with an amide being present under conditions such that amidase is induced in the bacterial cells. Suitably cultivation takes place at a temperature in the range 20° to 40°C, preferably 34° to 38°C, and at a pH in the range 5.0 to 8.0, preferably 5.8 to 7.6. Cultivation can be by batch culture, single stage continuous culture or multiple stage continuous culture. A suitable dilution rate for continuous culture is in the range 0.05 $hr^{-1}$ to 0.55 $hr^{-1}$. Methanol is the preferred carbon source. Any suitable amide may be present in the culture medium. Acetamide or formamide are preferred. Other suitable amides include amides of carboxylic acids having the formula:-

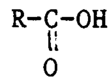

Where R is a short chain aliphatic group, i.e. containing 1 to 5 carbon atoms, which may be a straight or a branched chain. Preferably the amide is the sole or major nitrogen source and cultivation is by continuous culture under nitrogen limitation. A very suitable culture medium has the following composition:-

| Component | | Amount present/litre |
|---|---|---|
| Phosphoric acid | : | 1.6 ml |
| $MgSO_4.7H_2O$ | : | 1.912 g |
| $K_2SO_4$ | : | 0.952 g |
| $CuSO_4.5H_2O$ | : | 0.840 mg |
| $ZnSO_4.H_2O$ | : | 2.568 mg |
| $MnSO_4.4H_2O$ | : | 4.04 mg |
| $FeSO_4.7H_2O$ | : | 37.20 mg |
| Calcium formate | : | 0.173 g |

Nitrogen is supplied either from acetamide alone or from acetamide and ammonia. Cells are grown in nitrogen excess or nitrogen limitation at a range of cell concentrations.

Cells produced by continuous, batch or fed batch fermentation can be harvested by any suitable means preferably by ultrafiltration or centrifugation to produce a slurry having 10 - 25% by weight dry solids. Suitably this slurry is broken, e.g. by several freeze-thaw cycles or by mechanical breakage in a bead mill or french pressure device. Cell debris may then be removed by centrifugation leaving a crude cell-free extract containing amidase. Heat treatment may take place before or after this centrifugation but preferably before as this gives improved sedimentation of the product during centrifugation in addition to stimulating activity. This extract can if desired be further purified by anionic ion exchange chromatography and gel filtration. The cell free amidase preparations are suitably stored cool at 0° to 10°C or as frozen solutions or as freeze dried preparations prior to hydration and use.

In the process of the invention amidase can be used to reduce free acrylamide residues occurring in all types of acrylamide polymers, particularly latices of the three basic chemical types. When treating a latex an amidase solution is added to the latex and is dispersed through the latex by stirring or similar mixing techniques at a level between 1 and 10,000 units/kg latex preferably 100 - 2000 U/kg latex. Incubation of amidase with the latex results in conversion of free acrylamide to acrylic acid or a salt thereof. Incubation temperatures of 10°C to 100°C (particularly 30° to 80°C) are preferred. The latices are suitably treated over a pH range 3 to 10, preferably 5 to 7.

The amidase with increased activity produced in the method of the invention converts acrylamide into acrylic acid. This reaction can therefore be used as a means for producing acrylic acid or, when carried out in the presence of a suitable salt or alcohol, to produce salts or esters of acrylic acid.

The amidase when heated by the method of the invention above exhibits very high activity. This greatly extends the range of utility of polyacrylamide polymers.

The invention is illustrated by the following Examples:-

EXAMPLE 1

Heat stimulation of the amidase activity of cell extracts of Methylophilus methylotrophus AS-1.

A series of cell free extracts prepared from Methylophilus methylotrophus AS-1 in a citrate-phosphate buffer system at pH 6.0 and at a concentration of 30 mg protein per ml were heated in a water bath for periods of 1 and 2 hours at a series of temperatures between 30° and 70°C. Samples were withdrawn at various times and were assayed for amidase activity at 30°C. The substrate used in all cases was acrylamide at a concentration of 100 mM. The assays were performed at 30°C in a 0.1 M citrate: 0.2 M

phosphate buffer system at pH 6.0.

The results are shown in the Table and show increased activity under most of the conditions used with the highest increases being at 60 ° C. Increases were greater after 2 hours heating than after 1 hour.

Table

| Treatment | | Amidase Activity (U/ml)* | Stimulation |
|---|---|---|---|
| none | | 3.69 | - |
| heating for 1 hr @ | 30°C | 3.69 | 1 x |
| | 50°C | 5.91 | 1.6 x |
| | 60°C | 24.65 | 6.7 x |
| | 70°C | 14.48 | 3.9 x |
| heating for 2 hrs @ | 30°C | 3.76 | 1.02 x |
| | 50°C | 8.86 | 2.04 x |
| | 60°C | 40.06 | 10.8 x |
| | 70°C | 2.28 | 0.62 x |

* 1 unit is defined as 1 μ mole of $NH_3$ released per minute.

EXAMPLE 2

Cell-free extracts as described in Example 1 were incubated at 55 ° C, 60 ° C and 65 ° C respectively. Samples were taken at various times and assayed for amidase activity as described in Example 1. The results are set out in Figure 1 which is a graph of amidase activity (Units/ml) against activation time (mins) and show optimal stimulation of amidase activity at 60 ° C over a period of two hours. Incubation at lower temperatures results in slower activation. Incubation at higher temperatures, e.g. at 65 ° C, shows an initial stimulation of rate of activation compared to 60 ° C but a lower final amidase activity.

EXAMPLE 3

Removal of acrylamide from an anionic latex.

An anionic latex, Nalfloc type A 626 produced by the Nalfloc Co. of Cheshire, UK, pH adjusted to 5.9 at 60 ° C was treated by addition of an amidase solution produced by the method of the invention of 300 units per ml activity to a final level of 1000 units amidase per kg latex. Samples were withdrawn at varying times and were analysed for free acrylamide by high pressure liquid chromatography (HPLC).

The results are set out in Figure 2 which is a graph of free acrylamide (mM) against treatment time (mins) and show a reduction in free acrylamide level from 1.78 mM (126 ppm) to 0.02 mM (1.4 ppm) within 30 minutes.

EXAMPLE 4

Removal of acrylamide from a cationic latex.

A cationic latex, Nalfoc type 4625-SC, pH adjusted to 6.0 was treated as described in Example 3 with samples being analysed by HPLC.

The results are set out in Figure 3 which is a graph of free acrylamide (mM) against treatment time

(mins) and show a reduction in free acrylamide level from 2.2 mM (156 ppm) to 0.02 mM (1.4 ppm) within 45 minutes.

EXAMPLE 5

Removal of acrylamide from a non-ionic latex.

A non-ionic latex, Nalfloc type 8861-SC pH adjusted to 6.0 was treated as described in Example 3 with samples being analysed by HPLC.

The results are set out in Figure 4 which is a graph of free acrylamide (mM) against treatment time (mins) and show a reduction in free acrylamide level from 3.1 mM (220 ppm) to 0.15 mM (10.7 ppm) within 120 minutes.

EXAMPLE 6

A series of cell-free extracts prepared from Methylophus methylotrophus pH adjusted to pH 5, 6, 7 and 8 by buffer exchange through Pharmacia RD10 gel filtration columns was incubated at 0°C at these pH's. Samples were taken for amidase activity analysis at various times at pH 6.0 as described in Example 1.

The results are set out in Figure 5 which is a graph of amidase activity (units (U)/ml) against time of heating (mins) and show maximum stimulation of amidase at pH 7.0. Incubation at pH 5, 6 and 8 results in significantly less stimulation of activity.

EXAMPLE 7

A paste comprising whole and partially broken cells of Methylophilus methylotrophus produced according to the method of the invention and having a solids content of 10% was heated at 60°C. Samples were taken at various times for amidase activity analysis as described in Example 1.

The results are set out in Figure 6 which is a graph of amidase activity in units (U) per mg dry cell weight (DCW) against heating time (hrs) and show maximum stimulation of activity after 7 hours increasing from 0.06 U/mg DCW to 1.25 U/mg DCW.

**Claims**

**1.** A method for the production of an amidase enzyme of increased activity wherein the activity of the enzyme is significantly increased by heating microbial cells or extracts containing the enzyme to a temperature in the range 40° to 80°C for a sufficient period at a pH of 4 to 9.

**2.** A method according to claim 1 wherein the cells or extracts containing the enzyme are heated to a temperature in the range 55° to 65°C.

**3.** A method according to claim 2 wherein the cells or extracts containing the enzyme are heated to a temperature in the range 58° to 62°C.

**4.** A method according to any one of the preceding claims wherein the heating of the cells or extracts containing the enzyme takes place at a protein concentration in the range 0.1 to 200 mg/ml.

**5.** A method according to any one of the preceding claims wherein the cells or extracts containing the enzyme are heated for a period in the range 20 minutes to 10 hours.

**6.** A process for the decomposition of acrylamide in a medium containing it in which the medium is contacted with an enzyme capable of decomposing acrylamide under conditions suitable for the enzyme to decompose the acrylamide wherein the enzyme is an amidase enzyme of which the activity has been significantly increased by heating to a temperature in the range 40° to 80°C for a sufficient period at a pH of 4 to 9.

**7.** A process according to claim 6 wherein the enzyme is one which has been heated to a temperature in the range 40° to 80°C whilst it is contained in broken cells and/or is associated with cell débris.

8. A process according to claim 6 or claim 7 wherein the enzyme has been induced in a strain of Methylophilus methylotrophus.

9. A process for the production of acrylic acid or a salt or ester thereof in which a medium containing acrylamide is contacted with an enzyme capable of decomposing acrylamide to acrylic acid under conditions suitable for the enzyme to decompose the acrylamide to acrylic acid or a salt or ester thereof wherein the enzyme is an amidase enzyme of which the activity has been significantly increased by heating to a temperature in the range 40° to 80°C for a sufficient period at a pH of 4 to 9.

## Patentansprüche

1. Verfahren zur Herstellung eines Amidase-Enzyms mit erhöhter Aktivität **dadurch gekennzeichnet,** daß die Aktivität des Enzyms durch Erhitzen von mikrobiellen Zellen oder Extrakten, die das Enzym enthalten, auf eine Temperatur im Bereich von 40 bis 80°C während eines ausreichenden Zeitraumes bei einem pH von 4 bis 9 bedeutend erhöht wird.

2. Verfahren nach Anspruch 1 **dadurch gekennzeichnet,** daß die Zellen oder Extrakte, die das Enzym enthalten, auf eine Temperatur im Bereich von 55 bis 65°C erhitzt werden.

3. Verfahren nach Anspruch 2 **dadurch gekennzeichnet,** daß die Zellen oder Extrakte, die das Enzym enthalten, auf eine Temperatur im Bereich von 58 bis 62°C erhitzt werden.

4. Verfahren nach einem der vorhergehenden Ansprüche **dadurch gekennzeichnet,** daß das Erhitzen der Zellen oder Extrakte, die das Enzym enthalten, bei einer Proteinkonzentration im Bereich von 0,1 bis 200 mg/ml stattfindet.

5. Verfahren nach einem der vorhergehenden Ansprüche **dadurch gekennzeichnet,** daß die Zellen oder Extrakte, die das Enzym enthalten, während eines Zeitraumes im Bereich von 20 Minuten bis 10 Stunden erhitzt werden.

6. Verfahren zur Aufspaltung von Acrylamid in einem Medium, das dieses enthält, in dem das Medium mit einem Enzym kontaktiert wird, das fähig ist, Acrylamid unter Bedingungen aufzuspalten, die für das Enzym zur Aufspaltung des Acrylamids geeignet sind **dadurch gekennzeichnet,** daß das Enzym ein Amidase-Enzym ist, dessen Aktivität durch Erhitzen auf eine Temperatur im Bereich von 40 bis 80°C während eines ausreichenden Zeitraumes bei einem pH von 4 bis 9 bedeutend erhöht wurde.

7. Verfahren nach Anspruch 6 **dadurch gekennzeichnet,** daß das Enzym auf eine Temperatur im Bereich von 40 bis 80°C erhitzt wurde, während es in zerbrochenen Zellen enthalten ist und/oder mit Zellüberresten verbunden ist.

8. Verfahren nach Anspruch 6 oder 7 **dadurch gekennzeichnet,** daß das Enzym in einem Stamm von Methylophilus methylotrophus induziert wurde.

9. Verfahren zur Herstellung von Acrylsäure oder einem Salz oder Ester davon, in dem ein acrylamidhaltiges Medium mit einem Enzym kontaktiert wird, das fähig ist, Acrylamid zu Acrylsäure unter Bedingungen aufzuspalten, die für das Enzym zur Aufspaltung des Acrylamids zu Acrylsäure oder einem Salz oder Ester davon geeignet sind, **dadurch gekennzeichnet,** daß das Enzym ein Amidase-Enzym ist, dessen Aktivität durch Erhitzen auf eine Temperatur im Bereich von 40 bis 80°C während eines ausreichenden Zeitraumes bei einem pH von 4 bis 9 bedeutend erhöht wurde.

## Revendications

1. Procédé pour la production d'une enzyme de type amidase d'activité accrue, dans lequel l'activité de l'enzyme est notablement augmentée par chauffage d'extraits ou de cellules microbiens contenant l'enzyme à une température de l'ordre de 40° à 80°C pendant une période de temps suffisante à un pH de 4 à 9.

**2.** Procédé suivant la revendication 1, dans lequel les cellules ou les extraits contenant l'enzyme sont chauffés à une température de l'ordre de 55° à 65°C.

**3.** Procédé suivant la revendication 2, dans lequel les cellules ou les extraits contenant l'enzyme sont chauffés à une température de l'ordre de 58° à 62°C.

**4.** Procédé suivant l'une quelconque des revendications précédentes, dans lequel le chauffage des cellules ou des extraits contenant l'enzyme a lieu à une concentration de protéine de l'ordre de 0,1 à 200 mg/ml.

**5.** Procédé suivant l'une quelconque des revendications précédentes, dans lequel les cellules ou les extraits contenant l'enzyme sont chauffés pendant une période temps de l'ordre de 20 minutes à 10 heures.

**6.** Procédé pour la décomposition d'acrylamide dans un milieu le contenant selon lequel le milieu mis en contact avec une enzyme capable de décomposer l'acrylamide dans des conditions convenables pour que l'enzyme décompose l'acrylamide, dans lequel l'enzyme est une enzyme de type amidase dont l'activité a été notablement accrue par chauffage à une température de l'ordre de 40° à 80°C pendant une période de temps suffisante à un pH de 4 à 9.

**7.** Procédé suivant la revendication 6, dans lequel l'enzyme est une enzyme qui a été chauffée à une température de l'ordre de 40° à 80°C alors qu'elle était contenue dans des cellules brisées et/ou associée à des débris de cellules.

**8.** Procédé suivant la revendication 6 ou la revendication 7, dans lequel l'enzyme a été induite dans une souche de Methylophilus methylotrophus.

**9.** Procédé pour la production d'acide acrylique ou d'un sel ou ester de celui-ci selon lequel un milieu contenant de l'acrylamide libre est mis en contact avec une enzyme capable de décomposer l'acrylamide en acide acrylique dans des conditions convenables pour que l'enzyme décompose l'acrylamide en acide acrylique ou un sel ou ester de celui-ci, dans lequel l'enzyme est une enzyme de type amidase dont l'activité a été notablement accrue par chauffage à une température de l'ordre de 40° à 80°C pendant une période de temps suffisante à un pH de 4 à 9.

FIG. 1

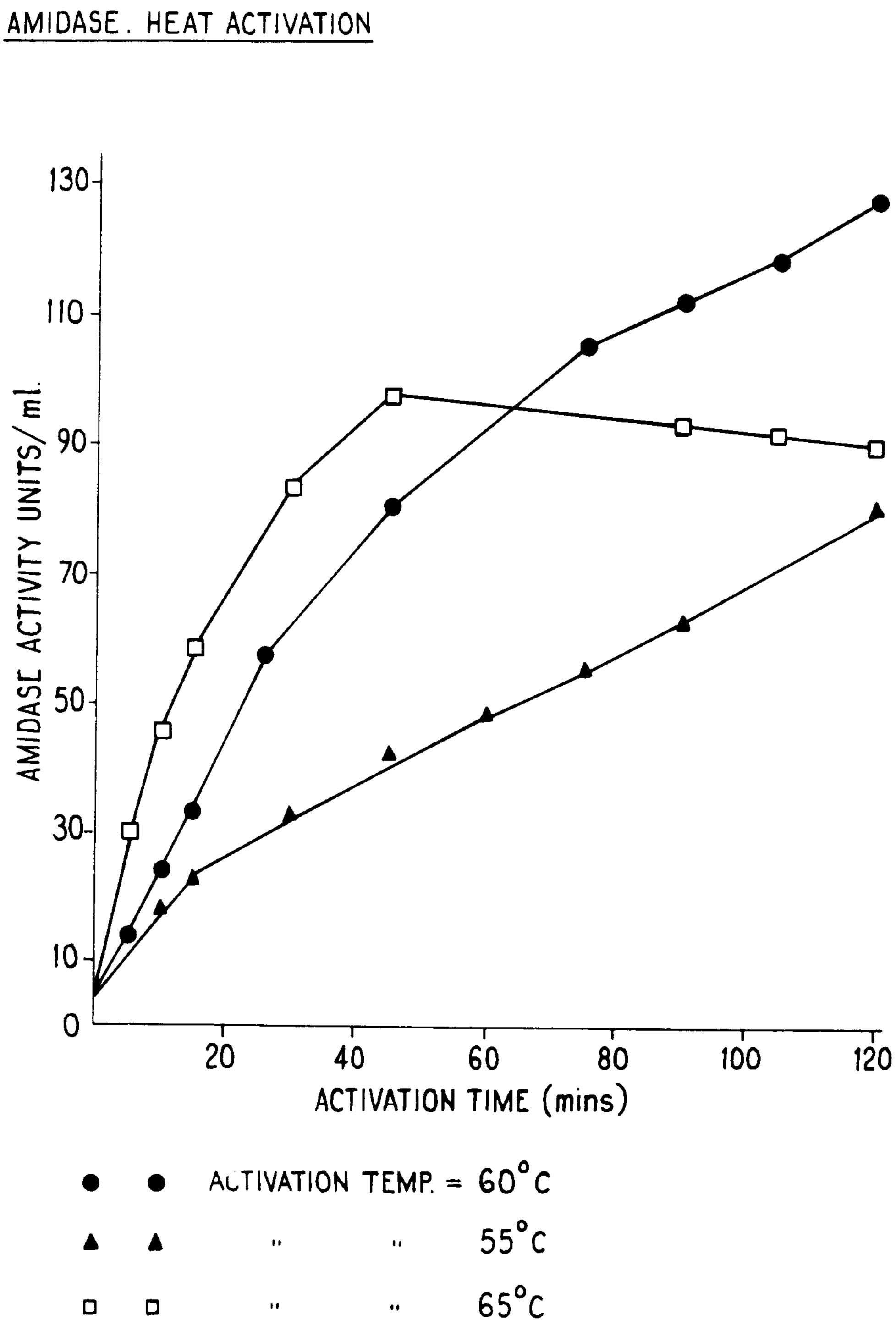
AMIDASE. HEAT ACTIVATION
AMIDASE ACTIVITY UNITS/ml.
130
110
90
70
50
30
10
0
20
40
60
80
100
120
ACTIVATION TIME (mins)
ACTIVATION TEMP. = 60°C
" " 55°C
" " 65°C

FIG. 2

AMIDASE. REMOVAL OF ACRYLAMIDE FROM AN ANIONIC LATEX TYPE A626

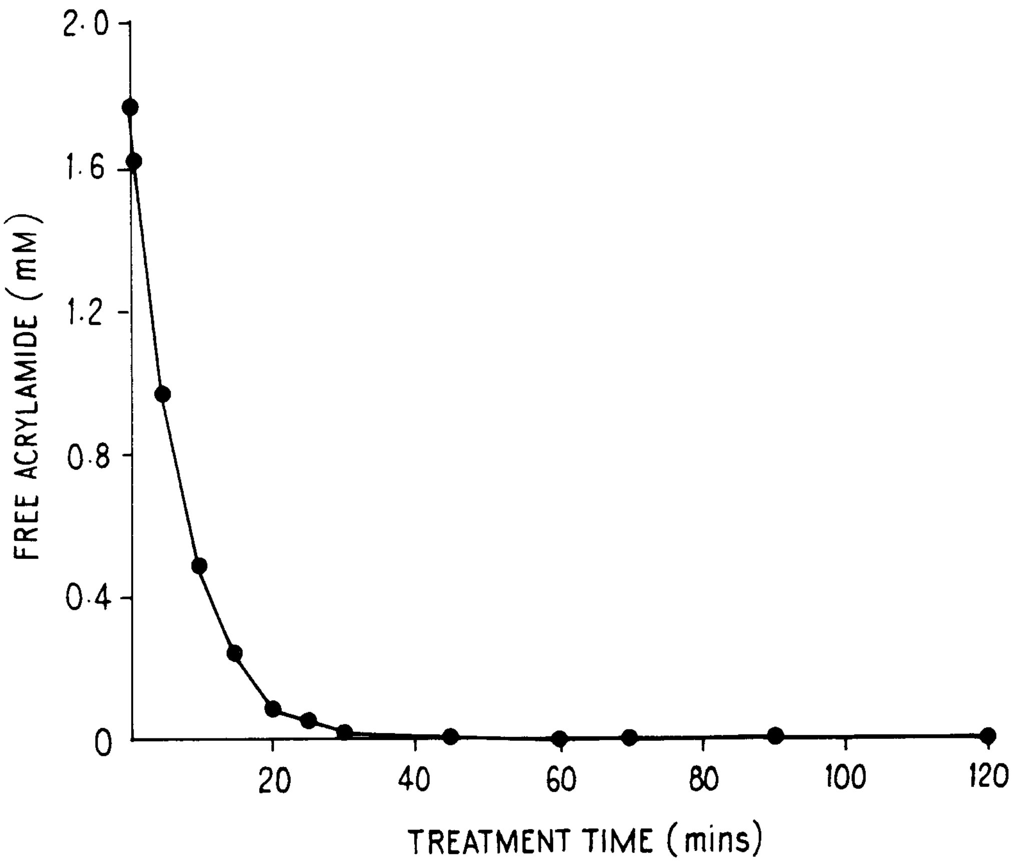


ANIONIC LATEX. NALFLOC TYPE A626
ENZYME LOADING 1000 U/Kg
TEMPERATURE 60°C
pH 5.9

FIG. 3

AMIDASE. REMOVAL OF ACRYLAMIDE FROM A CATIONIC LATEX TYPE 4625 - SC.

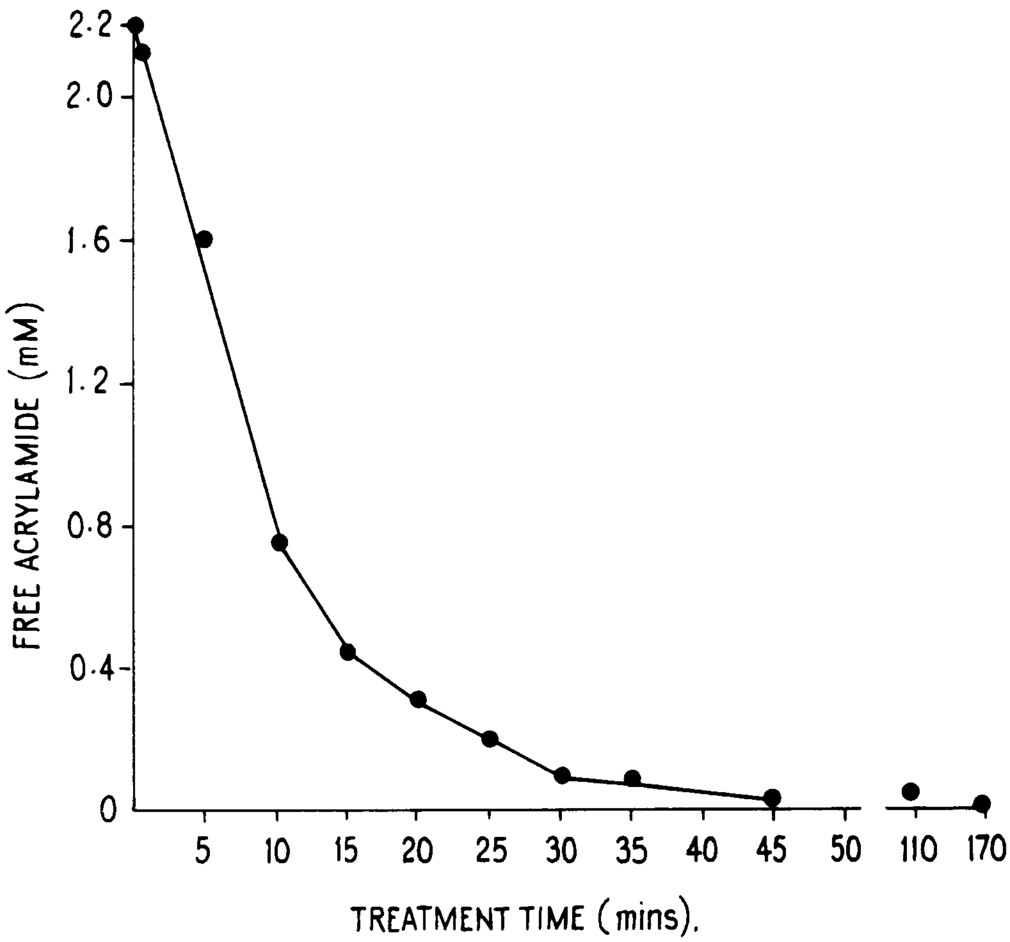


CATIONIC LATEX. NALFLOC TYPE 4625-SC
ENZYME LOADING 1000 U/Kg LATEX
pH 6.0

FIG.4

REMOVAL OF FREE ACRYLAMIDE FROM A NONIONIC LATEX
NALFLOC TYPE 8861-SC.

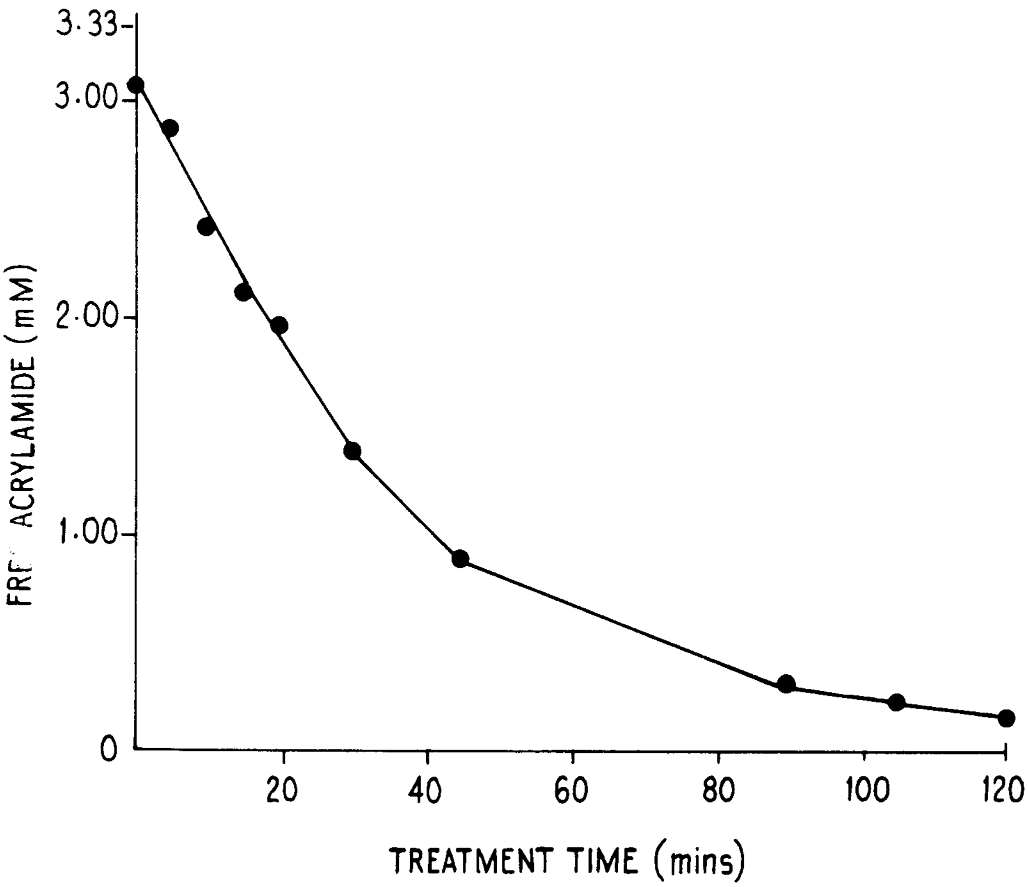


TEMPERATURE 60°C
pH 6·0
ENZYME LOADING 1000 U/Kg LATEX.

FIG.5

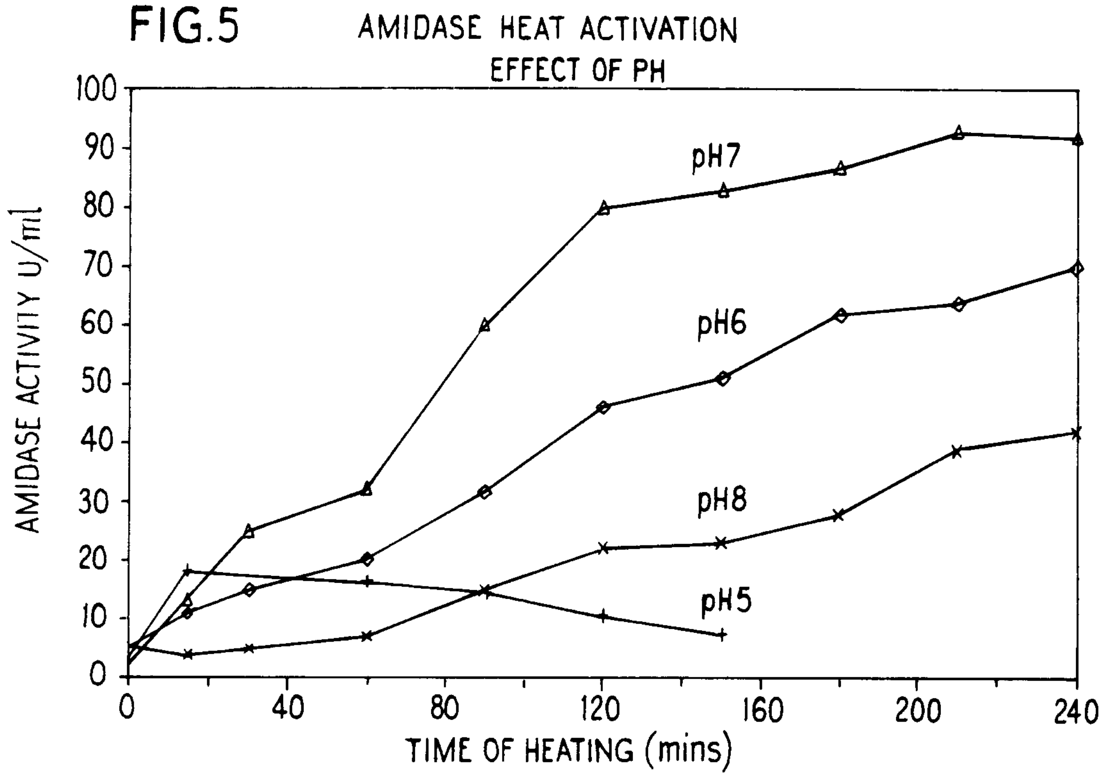
AMIDASE HEAT ACTIVATION
EFFECT OF PH
AMIDASE ACTIVITY U/ml
100
90
80
70
60
50
40
30
20
10
0
pH7
pH6
pH8
pH5
0
40
80
120
160
200
240
TIME OF HEATING (mins)

FIG.6

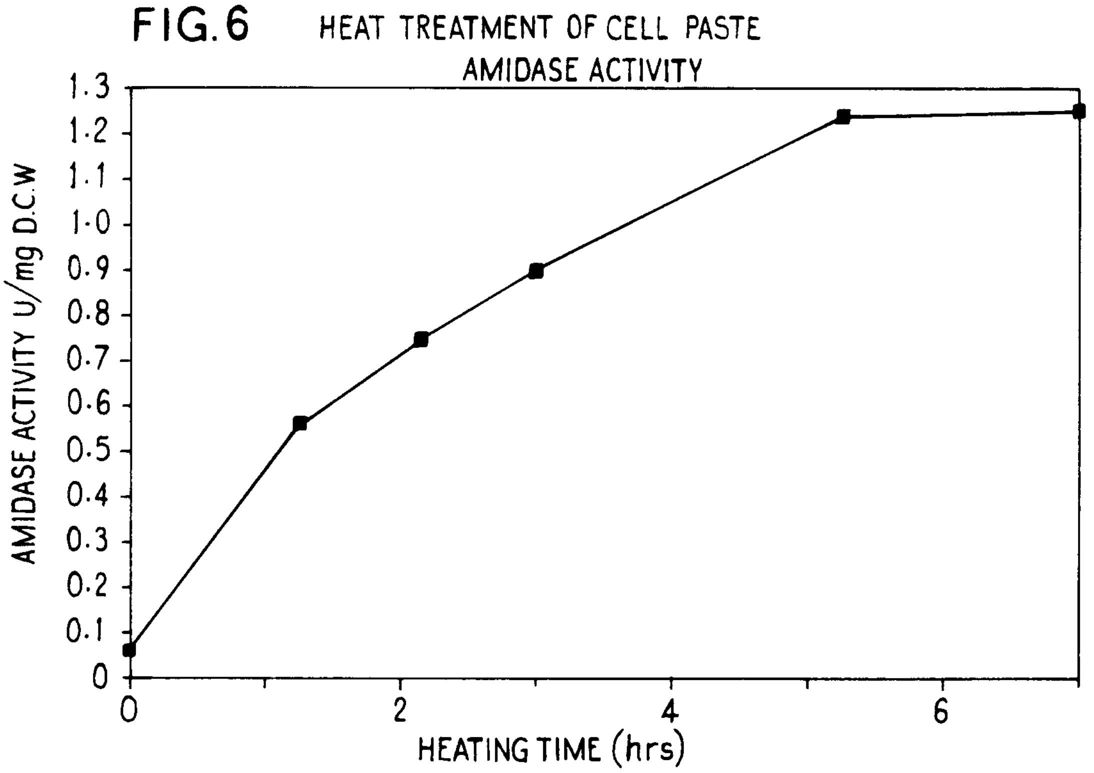
HEAT TREATMENT OF CELL PASTE
AMIDASE ACTIVITY
AMIDASE ACTIVITY U/mg D.C.W
1.3
1.2
1.1
1.0
0.9
0.8
0.7
0.6
0.5
0.4
0.3
0.2
0.1
0
0
2
4
6
HEATING TIME (hrs)